(19) Europäisches Patentamt
European Patent Office
Office européen des brevets

(11) Veröffentlichungsnummer: **0 017 699**
**B1**

(12) # EUROPÄISCHE PATENTSCHRIFT

(45) Veröffentlichungstag der Patentschrift: **16.06.82**

(51) Int. Cl.³: **C 07 C 143/78,** C 07 D 295/22, G 03 C 1/58

(21) Anmeldenummer: **80100287.4**

(22) Anmeldetag: **21.01.80**

(54) **Derivate des 2-Hydroxy-naphthalins und Diazotypiematerial, das diese als Kupplungskomponente enthält.**

(30) Priorität: **29.01.79 DE 2903342**

(43) Veröffentlichungstag der Anmeldung: **29.10.80 Patentblatt 80/22**

(45) Bekanntmachung des Hinweises auf die Patenterteilung: **16.06.82 Patentblatt 82/24**

(84) Benannte Vertragsstaaten: **AT BE CH DE FR GB IT NL SE**

(56) Entgegenhaltungen:
CH-A-223303
DD-B-26047
DE-A-2346459
DE-B-1077670
DE-B-2000819
DE-C-859027

Landau-Bericht, «Kupplungskomponenten», Heft 9 bis 17, 1962.

(73) Patentinhaber: **HOECHST AKTIENGESELLSCHAFT, Postfach 80 03 20, D-6230 Frankfurt/Main 80 (DE)**

(72) Erfinder: **Scheler, Siegfried, Dr., Stormstrasse 5, D-6200 Wiesbaden (DE)**

**EP 0 017 699 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Derivate des 2-Hydroxynaphthalins und Diazotypiematerial, das diese als Kupplugskomponente enthält.

Die Erfindung bezieht sich auf Derivate des 2-Hydroxynaphthalins, ihre Herstellung und ihre Verwendung als Kupplungskomponente in Diazotypiematerialien.

Verbindungen wie 2,3-Dihydroxynaphthalin und die Salze der 2,3-Dihydroxynaphthalin-6-sulfosäure, der 2-Hydroxynaphthalin-3,6-disulfosäure und der 2-Hydroxynaphthalin-3,7-disulfosäure sind bekannt, und sie werden zum Beispiel als Blaukuppler in der Diazotypie eingesetzt. Sie werden auch in Kombination mit gelb bzw. braun kuppelnden Komponenten zur Einstellung von schwarz kopierenden Diazotypiematerialien eingesetzt, die sich besonders als Zwischenoriginalmaterialien zum Weiterduplizieren eignen.

Ein Nachteil des 2,3-Dihydroxynaphthalins ist seine hohe Kupplungsaktivität, die bei unbelichteten Diazotypiematerialien zu unerwünschter Vorkupplung und damit zu einer geringen Haltbarkeit und Lagerfähigkeit führt. Nachteilig ist auch, dass von einer Vorlage mit unterschiedlichen optischen Dichten, einer sogenannten Halbtonvorlage, die zu erhaltende Diazokopie zu verschiedenfarbigen Dichtewerten entwickelt. Andererseits kuppeln die Salze der 2,3-Dihydroxynaphthalin-6-sulfosäure, der 2-Hydroxynaphthalin-3,6-disulfosäure und der 2-Hydroxynaphthalin-3,7-disulfosäure, von denen insbesondere die Natriumsalze Bedeutung erlangt haben, mit den in der Diazotypie üblichen Diazoverbindungen vom p-Phenylendiamintyp in der Regel zu langsam. Besonders gravierend ist dieser Nachteil dann, wenn ein Diazotypiematerial, das die langsam kuppelnden Komponenten enthält, bei normaler Temperatur mit trockenem Ammoniakgas unter Druck entwickelt werden soll. Bei der wegen ihrer Umweltfreundlichkeit favorisierten Druckgasentwicklung entwickeln solche Diazotypiematerialien nur zu sehr flauen, grünstichig grauen Farbtönen. Nachteilig ist auch, dass die Natriumsalze der 2,3-Dihydroxynaphthalin-6-sulfosäure, der 2-Hydroxynaphthalin-3,6-disulfosäure und der 2-Hydroxynaphtahalin-3,7-disulfosäure in organischen Lösungsmitteln praktisch unlöslich sind. Sie sind somit für eine Sensibilisierung aus organischer Phase, wie sie bei der Herstellung transparenter Diazotypiematerialien auf Folienbasis praktiziert wird, nicht geeignet.

Es ist auch bekannt, 2-Hydroxy-3-naphthoesäureamide (DE-AS Nr. 1178704) und in 6-Position durch Halogen oder Alkoxy substituierte 2-Hydroxy-3-naphthoesäureamide (DE-AS Nr. 1572078) als blau kuppelnde Komponenten in Diazotypiematerialien einzusetzen. Nachteil dieser Verbindungen ist die unerwünscht hohe optische Dichte des Bildhintergrundes im langwelligen ultravioletten und kurzwelligen visuellen Spektralbereich. Der gravierendste Nachteil dieser Blaukuppler ist jedoch die unzureichende Lichttechtheit ihrer Azofarbstoffe, die bei der Kupplung mit üblichen Diazoverbindungen gebildet werden. Eine hohe Lichtechtheit von Azofarbstoffen ist jedoch dann erwünscht, wenn eine Kopie, zum Beispiel eine technische Zeichnung, längere Zeit dem Sonnenlicht oder ein Diazozwischenoriginal, das für die Herstellung weiterer Diazokopien verwendet wird, dem aktinischen Licht handelsüblicher Pauslampen ausgesetzt wird.

Ganz besonders hohe Anforderungen an die Lichtechtheit von Diazotypieazofarbstoffen werden an Diazomikrofilmkopien gestellt. Solche Kopien werden zur Auswertung ihres Informationsgehaltes in Lese- und Leserückvergrösserungsgeräten einer besonders starken Strahlenbelastung — sichtbares und ultraviolettes Licht, Wärmestrahlung — durch die in diesen Geräten eingebauten Lichtquellen ausgesetzt. Es kommt häufig vor, dass Diazomikrofilmkopien, die längere Zeit der Emission der in Lesegeräten eingebauten Glühlampen ausgesetzt sind, so stark ausbleichen, dass ihre gespeicherten Informationen nur noch schwer lesbar sind.

Zur Verbesserung der Lichtechtheit von Diazotypie-Azofarbstoffen wurden auch bereits 2-Hydroxy-3-naphthoesäureamide mit einer Sulfonamidgruppe in 6-Position vorgeschlagen (europäische Patentanmeldung 79100723.0, Veröffentlichungsnummer 0004574). Diese Verbindungen kuppeln mit den in der Diazotypie üblichen Diazoverbindungen zu blauen Azofarbstoffen von guter Lichtechtheit. Allerdings ist die Kupplungsgeschwindigkeit dieser Verbindungen, insbesondere bei der Druckgasentwicklung und bei der konventionellen Entwicklung mit feuchtem Ammoniakgas bei niedrigen Temperaturen, für die Praxis nicht immer voll ausreichend.

Aus dem Landau-Bericht, „Kupplungskomponenten", Heft 9-17, 1962, sind 2,3-Dihydroxynaphthalin-6-sulfonamide und 2-Hydroxynaphthalin-3,6- und 3,7-disulfonamide bekannt, bei denen ein Wasserstoffatom oder beide der Sulfonamidgruppen durch Äthyl-, Hydroxyäthyl- oder Diäthylaminoäthylgruppen substituiert sind. Diese Verbindungen kuppeln zu rotstichig blauen bis violetten Azofarbstoffen von nur mässiger Lichtechtheit.

Ferner sind Diazotypiematerialien, vornehmlich zur Herstellung von Negativkopien, mit wasserunlöslichen Diazoiminoverbindungen bekannt, bei denen die lichtempfindliche Schicht alkalisch eingestellt ist und bei denen 2,3-Dihydroxynaphthalin-6-sulfanilid als Blaukuppler verwendet wird (US-PS Nr. 4055425). Im Falle der Anwendung dieses Verfahrens zur Herstellung von Positivkopien erweist es sich wegen der zusätzlichen Einstellung der Schicht auf einen sauren pH-Bereich als umständlich. Ausserdem kuppelt dieses Material bei konventioneller und besonders bei Druckgasentwicklung nicht ausreichend schnell.

Aufgabe der Erfindung war es deshalb, neue Verbindungen zur Verfügung zu stellen, die die Nachteile der oben genannten nicht oder nur in sehr viel geringerem Ausmasse aufweisen und die besonders hinsichtlich der Lichtechtheit ihrer Azofarbstoffe den bisher bekannten überlegen sind. Die gestellte Aufgabe wird gelöst durch Derivate des 2-Hydroxynaphthalins der allgemeinen Formel

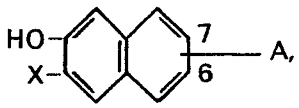

worin

A in 6- oder 7-Position 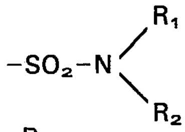

und

X –OH oder 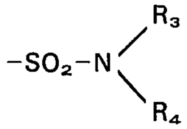

bedeuten, und $R_1$ und $R_3$ Wasserstoff, $R_2$ und $R_4$ gleich oder verschieden sind und, gegebenenfalls durch Niederalkylgruppen substutuiertes Cycloalkyl mit bis zu 8 Kohlenstoffatomen, Aralkyl mit bis zu 10 Kohlenstoffatomen oder durch Niederalkyl-, Niederalkoxyl-, Halogenalkyl-, alkylsubstituierte Aminogruppen oder durch Halogen substituiertes Aryl oder jeweils $R_1$ und $R_2$ bzw. $R_3$ und $R_4$ zusammen mit dem Stickstoffatom, an welches sie gebunden sind, gleiche oder verschiedene, gegebenenfalls Niederalkylsubstituierte heterocyclische Reste darstellen.

Besonders geeignet sind Derivate des 2-Hydroxynaphthalins, bei denen $R_2$ und $R_4$ gleich oder verschieden sind und Cycloalkyl mit bis zu 7 Kohlenstoffatomen, Aralkyl mit bis zu 10 Kohlenstoffatomen oder durch Alkyl-, Alkoxyl-, Halogenalkyl- oder Dialkylaminogruppen mit bis zu 4 Kohlenstoffatomen oder durch Halogen substituiertes Aryl mit bis zu 10 Kohlenstoffatomen darstellen, oder $R_1$ und $R_2$ bzw. $R_3$ und $R_4$ zusammen mit dem Stickstoffatom, an das sie gebunden sind, Pyrrolidin, Piperidin, Piperazin, Morpholin, Thiomorpholin oder Hexamethylenimin bedeuten.

Bevorzugt geeignet sind solche 2-Hydroxynaphthalinderivate, bei denen X eine Hydroxylgruppe ist. Verbindungen dieser Art eignen sich aufgrund ihrer erhöhten Kupplungsgeschwindigkeit besonders in Kombination mit langsamer kuppelnden Diazoverbindungen, wie sie üblicherweise in Zweikomponenten-Diazomaterialien verwendet werden. Dabei bewähren sich solche Derivate hervorragend, die einen Sulfonamidrest tragen, in welchem $R_1$ Wasserstoff und $R_2$ durch mindestens eine Niederalkyl-, Niederalkoxyl-, Halogenalkyl-, oder Dialkylaminogruppe oder durch Halogen substituiertes Phenyl, vorzugsweise durch mindestens eine Methyl- oder Trifluormethylgruppe oder durch Chlor substituiertes Phenyl bedeuten.

Geeignet sind auch solche Derivate des 2-Hydroxynaphthalins, bei denen X eine Sulfonamidgruppe bedeutet. Verbindungen dieser Art eignen sich aufgrund ihrer geringeren Kupplungsgeschwindigkeit besonders gut in Kombination mit schnell kuppelnden Diazoverbindungen, wie man sie üblicherweise in Einkomponenten-Diazotypiematerialien einsetzt. In diesen Verbindungen bedeuten $R_1$ und $R_3$ Wasserstoff und $R_2$ und $R_4$ sind gleich oder verschieden und stellen durch mindestens eine Niederalkyl-, Niederalkoxyl-, Halogenalkyl- oder Dialkylaminogruppe oder durch Halogen substituiertes Phenyl, vorzugsweise durch mindestens eine Methyl- oder Trifluormethylgruppe oder durch Chlor substituiertes Phenyl, dar.

Die neuen Verbindungen werden in der Diazotypie besonders dort vorteilhaft eingesetzt, wo Sensibilisierungsformulierungen auf Lösungsmittelbasis verwendet werden. Für den Einsatz in wässrigen Formulierungen sind solche Verbindungen der allgemeinen Formel bevorzugt, bei denen $R_1$ und $R_3$ Wasserstoff und $R_2$ und $R_4$ gleich oder verschiedenes Cyxloalkyl, Aralkyl oder Aryl sind, die durch salzbildende basische Reste, zum Beispiel die Dialkylaminogruppe, substituiert sind. Besonders bevorzugt sind solche Verbindungen der allgemeinen Formel, bei denen $R_1$ und $R_3$ Wasserstoff und $R_2$ und/oder $R_4$ Phenyl ist, das durch mindestens einen basischen Rest substituiert ist, der als solcher, als Salz, als Schwermetall-Komplexsalz oder als quaternäres Ammoniumsalz vorliegt. Die Salzbildung am basischen Rest kann in bekannter Weise mit jeder Säure erfolgen, die beständige Aminsalze bildet. Bevorzugt werden die Hydrochloride. Als komplexe Schwermetall-Aminsalze sind die Zinkchloridkomplexsalze bevorzugt. Ist der basische Rest quaterniert, so ist der vierte organische Rest vorzugsweise eine niedere Alkylgruppe. Der basische Rest kann auch gegebenenfalls ein alkylsubstituierter heterocyclischer Rest sein. Bevorzugt sind basische Heterocyclen mit 5-8 Ringgliedern, wie Pyrrolidin, Piperidin, Piperazin, Morpholin, Thiomorpholin oder Hexamethylenimin.

Beispiele für die erfindungsgemässen Derivate des 2-Hydroxynaphthalins, welche bevorzugt als Kupplungskomponenten in Diazotypiematerialien verwendet werden, sind, zusammen mit ihren Schmelz- bzw. Zersetzungstemperaturen der beigefügten Formelsammlung zu entnehmen.

Die erfindungsgemässen Derivate des 2-Hydroxynaphthalins können, beschrieben für die 2,3-Dihydroxynaphthalin-6-sulfonamide, auf folgendem Wege hergestellt werden:

2,3-Dihydroxynaphthalin-6-sulfosäure (Na-Salz), technisch unter Dinolsalz bekannt, wird mit Benzolsulfochlorid nach Schotten-Baumann bei 60-70°C und einem pH-Wert von 7-8 zu 2,3-Dibenzolsulfonyloxynaphthalin-6-sulfosäure (Na-Salz) umgesetzt. Dieses wird nach Isolierung und Trocknung mit überschüssigem Phosphoroxidchlorid in das entsprechende Sulfochlorid übergeführt. Das Reaktionsgemisch wird in Eiswasser eingerührt und das 2,3-Dibenzolsulfonyloxynaphthalin-6-sulfochlorid als farblose kristalline Substanz isoliert (Fp.: 151-152°C). Durch Umsetzung des Sulfochlorids mit den durch die Reste $R_1$ und $R_2$ substituierten Aminbasen in Toluol bei 100-115°C erhält man die entsprechenden 2,3-Dibenzolsulfonyloxynaphthalin-6-sulfonamide, die anfangs ölig anfallen, dann aber durch Behandlung mit verdünnter Salzsäure sehr schnell zu farblosen kristallinen Verbindungen erstarren. Die Abspaltung der Benzolsulfonylschutzgruppe erfolgt anschliessend durch kurzzeitiges Erhitzen in wässrig-alkalischer Lösung bei 80-90°C (Dampfbadtemperatur). Durch Ansäuern der alkalischen Lösung werden die 2,3-Dihydroxynaphthalin-6-sulfonamide ausgefällt, die zuerst ölig anfallen, aber nach kurzer Zeit durch Animpfen oder Reiben an der Gefässinnenwandung fest werden. (Ausbeute: 70-80% der Theorie, bezogen auf Dinolsalz).

Die Herstellung der 2-Hydroxynaphthalin-3,6- und 3,7-disulfonamide erfolgt auf analogem Wege, nur wird bei der Synthese dieser Verbindungen 2-Hydroxynaphthalin-3,6- bwz. 3,7-disulfosäure (Na-Salz) als Ausgangsmaterial eingesetzt. Das auf diesem Syntheseweg als Zwischenstufe anfallende 2-Benzolsulfonyloxynaphthalin-3,6-disulfochlorid schmilzt bei 188-190°C, das isomere 2-Benzolsulfonyloxynaphthalin-3,7-disulfochlorid bei 177-181°C. Durch Umsetzung der Sulfochloride mit den durch die Reste $R_1$ und $R_2$ bzw. $R_3$ und $R_4$ substituierten Aminbasen erhält man die entsprechenden 3,6- bzw. 3,7-Disulfonamide, die durch Abspaltung der Benzolsulfonylschutzgruppen in die 2-Hydroxynaphthalin-3,6- bzw. 3,7-disulfonamide übergeführt werden (Ausbeuten: 60-70% der Theorie, bezogen auf Ausgangsmaterial).

Als Träger für die Diazotypieschicht ist jede übliche Unterlage geeignet wie zum Beispiel beschichtete oder unbeschichtete, opake, transparente oder gefärbte Papiere, Textilien oder Kunststoffolien. Besonders geeignete Kunststoffolien sind solche aus Celluloseester, wie Cellulose-2 1/2- oder -triacetat, aus Polypropylen, aus Polyester, wie Polyäthylenterephthalat, oder aus Vinylpolymeren, wie Polyvinylacetat oder Polystyrol.

Die mit den erfindungsgemässen Kupplungskomponenten zur Erzeugung blauer Farbtöne verwendeten Diazoverbindungen sind bekannt. Es sind überwiegend Derivate des einseitig diazotierten p-Phenylendiamins mit mindestens einem Substituenten, zum Beispiel gegebenenfalls substituiertes Alkyl, Cycloalkyl, Aralkyl oder Aryl, an der Amingruppe. Bevorzugt sind solche Diazoverbindungen vom einseitig diazotierten p-Phenylendiamintyp, die in 2- und 5-Stellung zur Diazoniumgruppe durch einen Alkoxyrest substituiert sind und bei denen die Substituenten am Aminostickstoffatom, an das sie gebunden sind, zu einem heterocyclischen Ringsystem, zum Beispiel einem Morpholinring, verknüpft sind.

Die erfindungsgemässen Derivate des 2-Hydroxynaphthalins kuppeln mit den in der Diazotypie bekannten Diazoverbindungen zu kräftigen blauen Azofarbstoffen. Im Vergleich zu den bekannten blauen Azofarbstoffen zeichnen sich die neuen durch eine zum Teil erheblich bessere Lichtechtheit aus. Die besten Ergebnisse werden erzielt, wenn das Molverhältnis von Diazoverbindung und Kupplungskomponente zwischen (1-1,2) : 1, vorzugsweise 1,1 : 1, liegt. Ein solches Verhältnis ist insofern überraschend, als üblicherweise ein Überschuss an Kupplungskomponente eingesetzt wird. Werden diese üblichen Verhältnisse jedoch angewendet, so erzielt man weniger gute Lichtechtheiten. Lechtechtheiten in Abhängigkeit vom Diazokomponentenverhältnis waren bisher jedoch nicht bekannt.

Vorteilhaft gegenüber den 2-Hydroxy-3-naphthoesäureamiden und den in 6-Stellung durch ein Bromatom oder eine Methoxygruppe substituierten Derivaten ist die deutlich geringere Eigenfärbung der erfindungsgemässen Derivate und der damit verbundene bessere Bildhintergrund bei Diazokopien.

Ein weiterer Vorteil der erfindungsgemässen Derivate des 2-Hydroxynaphthalins, insbesondere der 2,3-Dihydroxynaphthalin-6-sulfonamide des neuen Verbindungstyps, gegenüber den bereits bekannten Salzen der 2,3-Dihydroxynaphthalin-6-sulfosäure und den 2-Hydroxy-3-naphthoesäureamiden-6-sulfonamiden ist ihre höhere Kupplungsgeschwindigkeit. Diese führt in Kombination mit den in Diazotypiematerialien bevorzugten Diazoverbindungen vom einseitig diazotierten p-Phenylendiamin zu einer schnelleren und vollständigeren Entwicklung. Der Vorteil der erhöhten Kupplungsgeschwindigkeit ist besonders dann von grösster anwendungstechnischer Bedeutung, wenn die Entwicklung von Diazotypiematerialien bei niedrigen Temperaturen mit trokkenem Ammoniakgas unter Druck oder mit feuchtem Ammoniakgas in konventionellen Dupliziergeräten durchgeführt werden soll. Durch die erhöhte Kupplungsgeschwindigkeit wird die Lagerfähigkeit des unbelichteten Diazotypiematerials nicht negativ beeinflusst. Die erfindungsgemässen Derivate des 2-Hydroxynaphthalins können sowohl allein als auch im Gemisch untereinander als Kupplungskomponenten in Diazotypiematerialien eingesetzt werden. Zur Erzielung anderer wünschenswerter Farbtöne, zum Beispiel schwarz, können sie auch im Gemisch mit andersfarbig kuppelnden Komponenten verwendet werden.

Dementsprechend richtet sich die vorliegende Erfindung auch auf ein Diazotypiematerial mit mindestens einer Diazoverbindung in der lichtempfindlichen Schicht, in welcher als Kupplungskomponente mindestens ein 2-Hydroxynaphthalin der allgemeinen Formel enthalten ist.

Die vorzugsweise für Zweikomponenten-Diazotypiematerialien verwendeten erfindungsgemässen Kupplungskomponenten werden zusammen mit der Diazokomponente in bekannter Weise aus wässriger, wässrig alkoholischer oder rein organischer Lösung auf den geeigneten Träger aufgebracht.

Werden Kunststoffolien als Trägermaterial verwendet, werden die Diazotypiebestandteile vorzugsweise aus einem organischen Medium, das ein filmbildendes Bindemittel enthält, in Form einer Lacklösung auf den Schichtträger aufgebracht. Die Konzentration der farbstoffbildenden Komponenten im Bindemittel kann variieren zwischen 15 und 30 Gewichtsteilen/100 Gewichtsteile Bindemittel. Beispiele für geeignete Bindemittel sind die verschiedensten polymeren Substanzen, z. B. Celluloseäther, wie Äthylcellulose,

sowie Celluloseester, wie Cellulose-2 1/2-acetat, -triacetat, -acetopropionat, -acetobutyrat, -butyrat, Vinylpolymerisate, wie Polyvinylacetat, Polyvinylidenchlorid, Vinylchlorid/Vinylacetat-Mischpolymerisate, Poly(methylmethacrylat)-Mischpolymerisate von Alkylacrylaten und Acrylsäure, sowie weitere Polymerisate, z. B. Polyphenylenoxid, Polyolefine, wie Polyäthylen oder Polypropylen sowie Äthylenglykol/Isophthalsäure/Terephthalsäure-Terpolymerisate

Die lichtempfindliche Schicht kann ausser den farbstoffbildenden Komponenten zusätzlich noch saure Stabilisatoren enthalten, wie Salzsäure, Borsäure, Zitronensäure, Weinsäure, Ameisensäure, und 5-Sulfosalicylsäure. Auch die in der Diazotypie üblichen Hilfsstoffe können in der lichtempfindlichen Schicht enthalten sein, z. B. anorganische Salze wie Zinkchlorid oder Ammonsulfat zur Kontraststeigerung, Harnstoff, Dimethylharnstoff, Äthylglykol, Äthylglykolmonomethyläther, Glycerin, Glycerindi- und triacetat als Kupplungsbeschleuniger, Thioharnstoff und Thioharnstoffderivate als Antivergilbmittel sowie Farbstoffe in kleinen Konzentrationen zur Anfärbung der ausbelichteten Bereiche der Diazokopie.

Die folgende Beispiele sollen die Erfindung näher erläutern

*Erläuterung zu den Beispielen:*

A. Der für die Herstellung der Diazotypiematerialien in den Beispielen 1-5 und 7-8 verwendete Basislack enthält 7,5 Gewichtsprozent Celluloseacetopropionat, gelöst in einem Lösungsmittelgemisch aus Aceton, Methanol, n-Butanol und Glykolmonomethyläther.

B. Die optischen Dichten der nach den Beispielen 1-5 und 7-8 hergestellten entwickelten Filmmuster werden mit einem Macbeth Quantalog-Densitometer TD 205 gemessen.

Für die Dichtemessungen im visuellen Spektralbereich ($D_{vis.}$) wird ein Filter, das im Bereich von 510-580 nm durchlässig ist (Kodak, Wratten Nr. 93), für die Dichtemessungen im ultravioletten Spektralbereich ($D_{uv}$) wird ein Filter, das im Bereich von 310-410 nm wirksam ist (Schott, UG 4), verwendet.

C. Zur Prüfung und Beurteilung der Lichtechtheit der bei der Volltonentwicklung entstandenen Diazotypiefarbstoffe der Beispiele 1, 7 und 8 wurde ein handelsübliches Lesegerät (Saul, LG 16F4) verwendet, das mit einer Halogenglühlampe von 15 V/150 W, einem Kühlaggregat und einem Objektiv für 24fache Vergrösserung ausgerüstet ist.

D. Belichtung und Entwicklung der nach den Beispielen 1-5 und 7-8 hergestellten Diazofilmmuster erfolgt in einem handelsüblichen Mikroplanfilm-Dupliziergerät (Kalle, Ozafiche).

Die Belichtung und Entwicklung des Diazotypiematerials des Beispiels 6 erfolgt in einem handelsüblichen Lichtpausgerät.

Die Entwicklung der Diazofilme des Beispiels 5 erfolgt in einem handelsüblichen Filmlochkarten-Dupliziergerät mit trockenem Ammoniakgas unter Druck.

*Beispiel 1*

Ausgehend von jeweils 50 ml Basislack werden 4 verschiedene Beschichtungslösungen der folgenden Zusammensetzung hergestellt.

| | |
|---|---|
| 90 mg | 5-Sulfosalicylsäure |
| 90 mg | Zinkchlorid |
| 290 mg | 2,5-Dibutoxy-4-morpholinobenzoldiazoniumfluoborat |
| $0{,}69 \times 10^{-3}$ | mol Blaukuppler, |

wobei als Blaukuppler verwendet werden:

1. 191 mg 2-Hydroxy-3-naphthoesäure-N(2-methylphenyl)amid (MG: 277)
2. 110 mg 2,3-Dihydroxynaphthalin (MG: 160)
3. 307 mg 2-Hydroxy-3-naphthoesäure-N(2-methylphenyl)amid-6-sulfonsäure-N(2-methylphenyl)amid (MG: 446)
4. 264 mg 2,3-Dihydroxynaphthalin-6-sulfonsäure-N(3-trifluormethylphenyl)-amid (MG: 383) Formel 13

Jede der Lösungen wird auf eine mit einer Haftschicht versehene Polyäthylenterephthalatfolie von 100 µ Dicke derart aufgetragen, dass die nach Trocknung im Umlufttrockenschrank bei 60-70°C erhaltenen verschiedenen Diazofilmmaterialien zu einer optischen visuellen Dichte von 1,60-2,20 entwickelbar sind. Die so hergestellten Diazofilmmaterialien werden entsprechend der verwendeten Sensibilisierungslösungen fortlaufend von 1-4 numeriert. Um eine vollständige Entwicklung zu gewährleisten, werden die Diazofilmmuster 1-4 jeweils zweimal entwickelt. Es ergeben sich folgende Farbtöne:

Diazofilmmuster 1: leicht rotstichig blau
Diazofilmmuster 2: violett
Diazofilmmuster 3: grünstichig blau
Diazofilmmuster 4: rotstichig blau

Anschliessend werden die zum Vollton entwickelten Diazofilme 12 h lang dem diffusen Tageslicht ausgesetzt.

Von jedem der so behandelten Volltonmuster wird ein Streifen von 0,5 cm × 5 cm Fläche abgeschnitten und dieser auf der Farbstoff tragenden Seite durch eine kreisförmige Messfläche von ca. 0,3 $cm^2$ markiert. In jeder Messfläche wird die optische visuelle Dichte ($D_{vis.}$) im grünen Spektralbereich (Kodak, Wratten-Filter Nr. 93) bestimmt.

Die markierten zum Vollton entwickelten Filmstreifen werden anschliessend der spektralen Emission der Lichtquelle eines handelsüblichen Lesegeräts (Saul, LG 16F4) gleichzeitig ausgesetzt. Nach jeweils 4stündiger Bestrahlungszeit bestimmt man in den Messflächen der Volltonmuster erneut die optische visuelle Dichte ($D_{vis., END}$) und vergleicht sie mit der Anfangsdichte ($D_{vis., ANF}$) Insgesamt werden die Volltonmuster 36 h lang im Lesegerät bestrahlt.

Zur Beurteilung der Lichtstabilität der Diazofilmmuster 1-4 werden ihre optischen visuellen Dichten zu Beginn (ANF) und zum Ende (END) in der Tabelle 1 zusammengestellt. In die Tabelle ist

mit aufgenommen die Halbwertszeit (τ) eines jeden Volltonmusters, d.h. die Zeit, nach der die optische visuelle Dichte auf die Hälfte ihres zu Beginn des Bestrahlungsversuches gemessenen Wertes abgefallen ist.

*Tabelle 1*

| Probe | $D_{vis.}$ ANF. | $D_{vis.}$ END. | Restdichte (%) | τ (h) |
|---|---|---|---|---|
| 1 | 2,06 | 0,05 | 2,5 | 5 |
| 2 | 2,24 | 0,20 | 9 | 11,5 |
| 3 | 1,62 | 0,34 | 21 | 18 |
| 4 | 1,77 | 0,90 | 50 | 36 |

Ein Vergleich der Dichtewerte zeigt sehr eindrucksvoll die überragende Lichtstabilität des Volltonmusters 4. Während der Azofarbstoff des Volltonmusters 4 nach 36 h Standzeit im Lesegerät noch 50% seiner ursprünglichen optischen Dichte aufweist, sind die optischen Dichten der Azofarbstoffe der Vergleichsmuster 2 und 3 bereits auf 9 bzw. 21%, die des Vergleichsmusters 1 sogar auf 2,5% ihrer ursprünglichen optischen Dichte abgefallen.

Vergleicht man die Halbwertszeiten τ der Volltonmuster 1-4, so erkennt man auch hier sehr deutlich die grosse Überlegenheit der erfindungsgemässen Probe 4 nach 36 h Standzeit im Lesegerät. Die Halbwertszeit des Volltonmusters 4 ist grösser gegenüber den Halbwertszeiten der Volltonmuster 1, 2 und 3 um die Faktoren 7 bzw. 3 und 2.

In Fig. 1 sind die gemessenen optischen Dichten (Ordinate) und die Halbwertszeiten (τ) der Volltonmuster 1-4 als Funktion der Bestrahlungszeit (t) des verwendeten Lesegerätes graphisch dargestellt.

*Beispiel 2*

Ausgehend von jeweils 50 ml Basislack werden 6 verschiedene Beschichtungslösungen der folgenden Zusammensetzung hergestellt:

| | |
|---|---|
| 90 mg | 5-Sulfosalicylsäure |
| 90 mg | Zinkchlorid |
| 260 mg | 2,5-Dibutoxy-4-morpholino-benzoldiazoniumfluoborat |
| $0,69 \times 10^{-3}$ | mol Blaukuppler, |

wobei als Blaukuppler verwendet werden:

5. 236 mg 2-Hydroxy-3-naphthoesäure-N(3-morpholinopropyl)amid (MG: 344)
6. 191 mg 2-Hydroxy-3-naphthoesäure-N(2-methylphenyl)amid (MG: 277)
7. 307 mg 2-Hydroxy-3-naphthoesäure-N(2-methylphenyl)amid-6-sulfonsäure-N(2-methylphenyl)amid (MG: 446)
8. 227 mg 2,3-Dihydroxynaphthalin-6-sulfonsäure-N(2-methylphenyl)amid (MG: 329), Formel 8
9. 332 mg 2-Hydroxynaphthalin-3,6-disulfonsäure-N(2-methylphenyl)amid (MG: 482), Formel 17
10. 332 mg 2-Hydroxynaphthalin-3,7-disulfonsäure-N(2-methylphenyl)amid (MG: 482), Formel 18

Von jeder Lösung wird, wie im Beispiel 1 beschrieben, ein Diazofilmmuster hergestellt. Anschliessend wird jeweils ein Streifen jedes Diazofilmmusters zum Vollton entwickelt. Ein weiterer Streifen eines jeden Diazofilmmusters wird vollständig ausbelichtet und anschliessend ebenfalls entwickelt. Von den so erhaltenen entwickelten Filmmustern werden die optischen Dichten im visuellen ($D_{vis.}$) und ultravioletten ($D_{UV}$) Spektralbereich bestimmt. Die gemessenen Dichtewerte sind in der Tabelle 2 zusammengestellt.

Muster 5 und 7 entwickeln zu grünstichig blauen, die Muster 6, 9 und 10 zu neutral blauen Farbtönen und das Muster 8 zu einem rotstichig blauen Farbton.

*Tabelle 2*

| Probe | Vollton $D_{vis.}$ | Vollton $D_{UV}$ | Bildhintergrund $D_{vis.}$ | Bildhintergrund $D_{UV}$ |
|---|---|---|---|---|
| 5 | 1,44 | 0,69 | 0,06 | 0,28 |
| 6 | 2,08 | 0,75 | 0,05 | 0,20 |
| 7 | 1,49 | 0,73 | 0,05 | 0,17 |
| 8 | 1,65 | 0,44 | 0,04 | 0,07 |
| 9 | 1,44 | 0,58 | 0,04 | 0,11 |
| 10 | 1,44 | 0,58 | 0,04 | 0,12 |

Die gemessenen Dichtewerte zeigen, dass die UV-Dichten des Bildhintergrundes der Filmmuster 8, 9 und 10 deutlich niedriger sind als die der Vergleichsmuster 5, 6 und 7. Die bei den Mustern 8, 9 und 10 verwendeten Blaukuppler gemäss der Erfindung sind deshalb als Stellkomponenten zur Herstellung von Zwischenoriginalmaterialien besser geeignet als die der Muster 5, 6 und 7.

*Beispiel 3*

Ausgehend von jeweils 50 ml Basislack werden 3 verschiedene Lösungen hergestellt:

| | |
|---|---|
| 90 mg | 5-Sulfosalicylsäure |
| 90 mg | Zinkchlorid |
| 260 mg | 2,5-Dibutoxy-4-morpholino-benzoldiazoniumfluoborat |
| $0,69 \times 10^{-3}$ | mol Blaukuppler, |

Folgende Blaukuppler werden verwendet:

11. 287 mg 2-Hydroxy-3-naphthoesäure-N(phenyl)amid-6-sulfonsäure-N(phenyl)amid (MG: 418)
12. 214 mg 2,3-Dihydroxynaphthalin-6-sulfonsäure-N-morpholid (MG: 309), Formel 2
13. 240 mg 2,3-Dihydroxynaphthalin-6-sulfonsäure-N(4-chlorphenyl)amid (MG: 350), Formel 11

Von jeder Lösung wird, wie im Beispiel 1 beschrieben, ein Diazofilmmuster hergestellt, entsprechend der verwendeten Lösungen fortlaufend von 11-13 numeriert und ein Streifen eines jeden Filmmusters von ca. 5 cm × 6 cm Fläche im Entwicklerteil eines handelsüblichen Planfilm-Dupliziergerätes einmal entwickelt.

Unmittelbar nach Verlassen des Entwicklerteils wird die UV-Dichte ($D_{UV, ANF}$) des erhaltenen Volltonmusters gemessen. Durch das Messlicht wird der Anteil der Diazoverbindung, der nicht zum Azofarbstoff gekuppelt wurde, photolytisch zerstört und damit die UV-Dichte erniedrigt. Je kleiner die Differenz $D_{UV, ANF} - D_{UV, END}$ ($\Delta D$) ist, um so grösser ist die Entwicklungsfreudigkeit des jeweiligen Filmmaterials, d.h. die Differenz $\Delta D$ ist ein Mass für die Entwicklungsgeschwindigkeit und dieser umgekehrt proportional.

Zur Beurteilung der Entwicklungsgeschwindigkeit der Filmmuster 11-13 sind in der Tabelle 3 die UV-Dichten $D_{UV, ANF}$ und $D_{UV, END}$ sowie die Dichtedifferenzen $\Delta D$ zusammengestellt.

*Tabelle 3*

| Probe | UV-Dichten | | $\Delta D$ |
|---|---|---|---|
| | $D_{UV, ANF}$ | $D_{UV, END}$ | |
| 11 | 0,98 | 0,64 | 0,34 |
| 12 | 0,54 | 0,53 | 0,01 |
| 13 | 0,42 | 0,39 | 0,03 |

Vergleicht man die Dichtedifferenzen $\Delta D$ der Proben 11-13 so erkennt man sehr deutlich, dass bei den Filmmustern 12 und 13 die Farbstoffkupplung praktisch vollständig abgelaufen ist, während das Vergleichsmuster 11, mit einem bekannten Blaukuppler in der lichtempfindlichen Schicht, eine wesentlich grössere Restdiazomenge enthält. Dies bedeutet, dass die Filmmuster 12 und 13 erheblich schneller entwickeln als das Vergleichsmuster 11. Prinzipiell ähnlich verhalten sich alle in der Formelsammlung aufgeführten erfindungsgemässen Verbindungen.

*Beispiel 4*

Eine mit einer Haftschicht versehene Polyäthylenterephthalatfolie wird einseitig auf der substrierten Oberfläche mit einer Lösung folgender Zusammensetzung beschichtet:

| | |
|---|---|
| 50 ml | Basislack |
| 90 mg | 5-Sulfosalicylsäure |
| 90 mg | Zinkchlorid |
| 332 mg | 2-Hydroxynaphthalin-3,6-disulfonsäure-N(2-methylphenyl)amid, Formel 17 |
| 250 mg | 2,5-Diäthoxy-4(4'-methylphenylmercapto)benzoldiazoniumfluoborat |

Nach dem Trocknen wird die sensibilisierte Folie unter einer transparenten Silberbildvorlage belichtet und in einem handelsüblichen Planfilm-Dupliziergerät (Kalle, Ozafiche) entwickelt. Man erhält eine kontrastreiche Filmkopie der Vorlage mit rotstichig blauen Linien auf klarem transparentem Hintergrund.

Das unbelichtete Diazofilmmaterial besitzt eine gute Lagerfähigkeit.

*Beispiel 5*

Ausgehend von jeweils 50 ml Basislack werden 2 verschiedene Beschichtungslösungen der folgenden Zusammensetzung hergestellt:

| | |
|---|---|
| 90 mg | 5-Sulfosalicylsäure |
| 90 mg | Zinkchlorid |
| 290 mg | 2,5-Dibutoxy-4-morpholinobenzoldiazoniumfluoborat |
| $0{,}69 \times 10^{-3}$ | mol Blaukuppler, |

wobei als Blaukuppler verwendet werden:

14. 264 mg 2,3-Dihydroxynaphthalin-6-sulfonsäure-N(3-trifluormethylphenyl)amid, Formel 13
15. 230 mg 2,3-Dihydroxynaphthalin-6-sulfonsäure (Anilinsalz)

Von jeder Lösung wird, wie im Beispiel 1 beschrieben, ein Diazofilmmuster mit der fortlaufenden Numerierung 14-15 hergestellt. Anschliessend werden von jedem dieser beiden Filmmuster jeweils 2 kleine Teilstücke von ca. 3 cm × 5 cm Fläche abgeschnitten. Die Abschnitte werden mit 14a und 14b bzw. 15a und 15b bezeichnet. 14a und 15a werden mit feuchtem Ammoniakgas bei ca. 60-70°C, 14b und 15b mit trockenem Ammoniakgas bei ca. 20-25°C unter Druck entwikkelt. Die bei den unterschiedlichen Entwicklungsbedingungen erzielten optischen visuellen Dichten und Farbtöne sind in der Tabelle 4 zusammengestellt.

*Tabelle 4*

| Probe | $NH_3$-Gas, feucht 60-70°C | | $NH_3$-Gas, trocken (Druck) 20-25°C | |
|---|---|---|---|---|
| | $D_{vis.}$ | Farbton | $D_{vis.}$ | Farbton |
| 14a | 1,60 | rotstichig blau | — | — |
| 14b | — | — | 1,45 | neutral blau |
| 15a | 1,17 | neutral blau | — | — |
| 15b | — | — | 0,58 | grünstichig grau |

Die Entwicklungsergebnisse zeigen, dass das Filmmuster 15 bei unterschiedlichen Entwicklungsbedingungen zu deutlich unterschiedlichen optischen visuellen Dichten und Farbtönen entwickelt. Das Filmmuster 14 mit den erfindungsgemässen Verbindungen hingegen zeigt diese Unterschiede in deutlich geringerem Ausmasse.

*Beispiel 6*

Auf ein blau eingefärbtes naturtransparentes, mit Celluloseacetat (ca. 50-54% Essigsäure) lackiertes Papier (Lackauftrag 10-20 g/m²) wird folgende Lösung aufgebracht, der Überschuss in geeigneter Weise entfernt und das Material getrocknet:

| | |
|---|---|
| 32,5 ml | Isopropanol |
| 12,5 ml | Wasser |

2,5 ml Ameisensäure
1,25 g Zitronensäure
0,75 g Borsäure
1,5 g Thioharnstoff
1,55 g 2,3-Dihydroxynaphthalin-6-sulfonsäure-N(4-diäthylaminophenyl)amid, Formel 16
1,53 g 2,5-Diäthoxy-4-morpholinobenzoldiazoniumchlorid (Zinkchlorid-Doppelsalz)

Nach Belichten unter einer Vorlage ergibt die Entwicklung mit feuchtem Ammoniakgas sehr kontrastreiche, rotstichig blaue Linien gei sehr guter Transparenz des Pausengrundes.

*Beispiel 7*

Eine beidseitig mit einer Haftschicht versehene Polyäthylenterephthalatfolie wird auf der einen Seite mit einer Lösung von

1,0 g Fettgelb 5G (C. I. Solvent Yellow 68) in
100 ml Basislack

beschichtet. Man trocknet bei 60-70°C im Umlufttrockenschrank und erhält einen Verbund aus Trägerfolie und gelber Beschichtung. Das Trockenschichtgewicht liegt bei etwa 6 g/m².

Nach der Trocknung wird die andere Folienseite mit einer Lösung folgender Zusammensetzung sensibilisiert:

120 ml Basislack
240 mg 5-Sulfosalicylsäure
400 mg N,N-Dimethylharnstoff
540 mg 2,3-Dihydroxynaphthalin-6-sulfonsäure-N(4-chlorphenyl)amid, Formel 11
660 mg 2,5-Dibutoxy-4-morpholinobenzoldiazoniumfluoborat

Nach erneuter Trocknung im Umlufttrockenschrank bei 60-70°C wird die so hergestellte sensibilisierte Folie unter einer transparenten Silberbildvorlage belichtet und einwickelt. Man erhält eine sehr kontrastreiche zweifarbige Kopie der Vorlage mit einem blauen Azofarbstoffbild auf der einen und einem gelben Bildhintergrund auf der anderen Seite der Folie. Durch einen Filmprojektor oder in einem Mikrofilmlesegerät sieht man ein grünstichig schwarzes Farbbild auf gelbem Hintergrund. Die Lesbarkeit der Filmkopie ist sehr gut. Verwendet man anstelle des erfindungsgemässen Blaukupplers die äquivalente Menge des bekannten Blaukupplers 2-Hydroxy-3-naphthoesäure-N(2-methylphenyl)amid in der lichtempfindlichen Schicht, dann erhält man nach der bildmässigen Belichtung auch eine zweifarbige Filmkopie. Beim Betrachten im Filmprojektor oder in einem Mikrofilmlesegerät sieht man ein grünstichig blaues Farbbild auf gelbem Hintergrund, dessen Kontrast und Lesbarkeit jedoch geringer ist als bei Verwendung des erfindungsgemässen Blaukupplers.

Von beiden Diazofilmkopien lässt sich keine weitere Kopie auf Diazomaterial anfertigen.

Vergleicht man die Lichtstabilitäten der beiden Filmkopien so erkennt man, dass die Filmkopie mit dem erfindungsgemässen Blaukuppler nach 12-stündiger Standzeit erheblich weniger ausbleicht und deshalb auch noch wesentlich besser lesbar ist als die Vergleichskopie.

Zur Beurteilung der besseren Lichtstabilität wird ein Filmmuster (Probe 17), das den erfindungsgemässen Blaukuppler enthält und ein Filmmuster (Probe 18), das den zu Vergleichszwecken verwendeten Blaukuppler enthält, zum Vollton entwickelt. Jeweils ein Abschnitt dieser beiden Volltonmuster wird dann gleichzeitig in einem Mikrofilmlesegerät während 12 h der spektralen Emission der Lichtquellen ausgesetzt.

In der folgenden Tabelle 5 sind die optischen Dichtewerte am Anfang und am Ende der Bestrahlungszeit zusammengestellt.

*Tabelle 5*

| Probe | $D_{vis.}$ ANF. | END. | Restdichte (%) | $\tau$ (h) |
|---|---|---|---|---|
| 17 | 1,60 | 0,80 | 50 | 12 |
| 18 | 1,51 | 0,24 | 16 | 4 |

Vergleich man die Halbwertszeiten der Volltonmuster 17 und 18, so sieht man, dass das Volltonmuster 17 erst nach 12 h, das Vergleichsmuster 18 jedoch bereits nach 4 h Standzeit im Lesegerät auf die Hälfte seines ursprünglichen Dichtewertes abgefallen ist. Das Volltonmuster 17 ist deshalb im Lesegerät um den Faktor 3 lichtstabiler als das Vergleichsmuster 18.

*Beispiel 8*

Eine mit einer Haftschicht versehene Polyäthylenterephthalatfolie wird einseitig auf der substrierten Oberfläche mit einer Lösung folgender Zusammensetzung beschichtet:

100 ml Basislack
410 mg 5-Sulfosalicylsäure
168 mg 2-Methylresorcin
33 mg 2,2',4,4'-Tetrahydroxydiphenylsulfid
200 mg Glycerintriacetat
168 mg Zinkchlorid
60 mg N,N-Dimethylharnstoff
320 mg 2,3-Dihydroxynaphthalin-6-sulfonsäure-N(4-chlorphenyl)amid, Formel 11
726 mg 2,5-Dibutoxy-4-morpholinobenzoldiazoniumfluoborat
77 mg 4-Dipropylaminobenzoldiazoniumfluoborat

Nach der Trocknung wird die beschichtete Folie unter einer transparenten Silberbildvorlage belichtet und entwickelt. Man erhält eine sehr kontrastreiche Filmkopie der Vorlage mit schwarzen Linien auf klarem transparenten Hintergrund. Die Filmkopie zeigt in handelsüblichen Mikrofilmlesegeräten eine hervorragende Lichtstabilität.

Verwendet man anstelle des erfindungsgemässen Blaukupplers nach Formel 11 eine äquivalente Menge 6-Methoxy-2-hydroxy-3-naphthoesäure-N(3-morpholinopropyl)amid gemäss deutscher Patentanmeldung P Nr. 2746551.6 in der lichtempfindlichen Schicht, dann erhält man auch eine Kopie mit schwarzen Linien auf leicht gelb gefärbtem transparenten Hintergrund. Die Lichtstabilität dieser Kopie im Mikrofilmlesegerät ist jedoch deutlich geringer als die mit der erfindungsgemässen Verbindung hergestellte Filmkopie.

Zur Demonstration der besseren Lichtstabilität der Filmbilbfarbstoffe im Lesegerät gegenüber bekannten schwarz duplizierenden Diazofilmen wird ein Filmmuster (Probe 19), das den Erfindungsgemässen Blaukuppler gemäss Formel 11 enthält und ein Filmmuster (Probe 20), das den Blaukuppler zu Vergleichszwecken enthält, zum Vollton entwickelt. Abschnitte dieser beiden Volltonmuster werden dann gleichzeitig in einem Lesegerät während 12 h bestrahlt. In der folgenden Tabelle 6 sind die optischen Dichtewerte der Volltonmuster 19 und 20 am Anfang und am Ende der Bestrahlungszeit zusammengestellt.

*Tabelle 6*

| Probe | $D_{vis.}$ ANF. | $D_{vis.}$ END. | Restdichte (%) | $\tau$ (h) |
|---|---|---|---|---|
| 19 | 1,56 | 1,25 | 80 | 30 |
| 20 | 1,55 | 0,28 | 18 | 6 |

Vergleich man die Restdichten der Volltonmuster 19 und 20, so erkennt man, dass das Volltonmuster 19 nach 12 h Standzeit im Lesegerät noch 80% seiner ursprünglichen optischen Dichte besitzt, das Volltonmuster 20 nach der gleichen Zeit jedoch nur noch 18%. Das Volltonmuster 19 ist deshalb nach 12 h Standzeit im Lesegerät um dem Faktor 4,5 lichtstabiler als das Vergleichsmuster 20.

*Formeln*

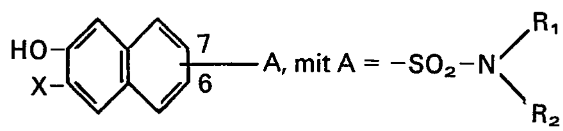

| Nr. | X | A (in Position) | $R_1$ | $R_2$ | Fp. (°C) |
|---|---|---|---|---|---|
| 1 | HO | 6 | H | 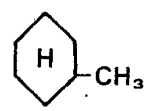 | 68- 70 |
| 2 | HO | 6 | 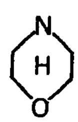 | | 218-220 |
| 3 | HO | 6 | 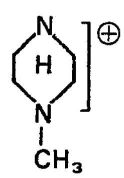 | 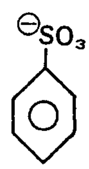 | >200° (Z.) |
| 4 | HO | 6 | H | 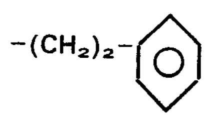 | 77- 80 |
| 5 | HO | 6 | H | 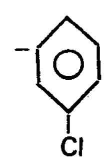 | 168-170 |
| 7 | HO | 6 | H | 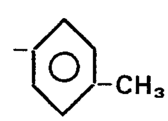 | 178-179 |
| 8 | HO | 6 | H | 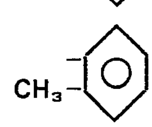 | 204-205 |
| 9 | HO | 6 | H | 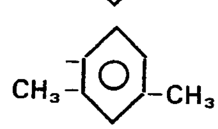 | 216-218 |

| Nr. | X | A (in Position) | $R_1$ | $R_2$ | Fp. (°C) |
|---|---|---|---|---|---|
| 10 | HO | 6 | H | 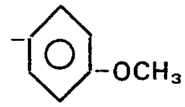 | 192-193 |
| 11 | HO | 6 | H | 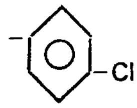 | 197-198 |
| 12 | HO | 6 | H | 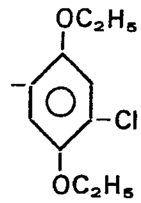 | 188-190 |
| 13 | HO | 6 | H | 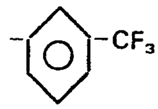 | 201-202 |
| 14 | HO | 6 | H | 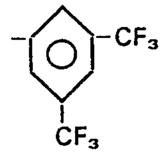 | 177-180 |
| 15 | HO | 6 | H | 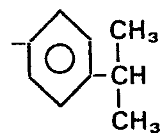 | 186-188 |
| 16 | HO | 6 | H | 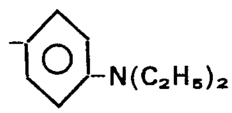 | 191-195 |
| 17 | 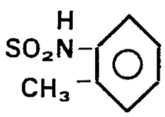 | 6 | H | 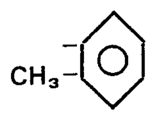 | 166-167 |
| 18 | 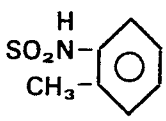 | 7 | H | 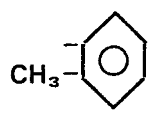 | 229-230 |
| 19 | HO | 6 | H | 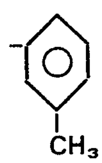 | 268-270 |

**Patentansprüche**

1. Derivate des 2-Hydroxynaphthalins der allgemeinen Formel

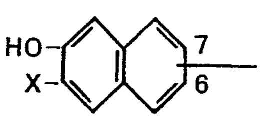 A,

worin
A in 6- oder 7-Position 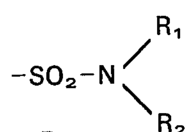
und
X -OH oder 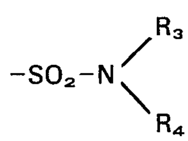
bedeuten, und $R_1$ und $R_3$ Wasserstoff, $R_2$ und $R_4$ gleich oder verschieden sind und, gegebenenfalls

durch Niederalkylgruppen substutuiertes Cycloalkyl mit bis zu 8 Kohlenstoffatomen, Aralkyl mit bis zu 10 Kohlenstoffatomen oder durch Niederalkyl-, Niederalkoxyl-, Halogenalkyl-, alkylsubstituierte Aminogruppen oder durch Halogen substituiertes Aryl oder jeweils $R_1$ und $R_2$ bzw. $R_3$ und $R_4$ zusammen mit dem Stickstoffatom, an welches sie gebunden sind, gleiche oder verschiedene, gegebenenfalls niederalkylsubstituierte heterocyclische Reste darstellen.

2. 2-Hydroxynaphthalin nach Anspruch 1, dadurch gekennzeichnet, dass $R_2$ und $R_4$ gleich oder verschieden sind und Cycloalkyl mit bis zu 7 Kohlenstoffatomen, Aralkyl mit bis zu 10 Kohlenstoffatomen oder durch Alkyl-, Alkoxyl-, Halogenalkyl- oder Dialkylaminogruppen mit bis zu 4 Kohlenstoffatomen oder durch Halogen substituiertes Aryl mit bis zu 10 Kohlenstoffatomen, oder $R_1$ und $R_2$ bzw. $R_3$ und $R_4$ zusammen mit dem Stickstoffatom, an das sie gebunden sind, Pyrrolidin, Piperidin, Piperazin, Morpholin, Thiomorpholin oder Hexamethylenimin bedeuten.

3. 2-Hydroxynaphthalin nach Anspruch 1, dadurch gekennzeichnet, dass X− eine Hydroxylgruppe bedeutet.

4. 2-Hydroxynaphthalin nach den beiden Ansprüchen 1 und 3, dadurch gekennzeichnet,dass A einen Sulfonamidrest darstellt, wobei $R_1$ Wasserstoff und $R_2$ durch mindestens eine Niederalkyl-, Niederalkoxyl-, Halogenalkyl- oder Dialkylaminogruppe und/oder durch Halogen substituiertes Phenyl bedeuten.

5. 2-Hydroxynaphthalin nach Anspruch 4, dadurch gekennzeichnet, dass $R_1$ Wasserstoff und $R_2$ durch mindestens eine Methyl- oder Trifluormethylgruppe und/oder durch Chlor substituiertes Phenyl bedeuten.

6. 2-Hydroxynaphthalin nach Anspruch 1, dadurch gekennzeichnet, dass X− eine Sulfonamidgruppe bedeutet.

7. 2-Hydroxynaphthalin nach den beiden Ansprüchen 1 und 6, dadurch gekennzeichnet, dass $R_1$ und $R_3$ Wasserstoff und $R_2$ und $R_4$ gleich oder verschieden sind und durch mindestens eine Niederalkyl-, Niederalkoxyl-, Halogenalkyl- oder Dialkylaminogruppe und/oder durch Halogen substituiertes Phenyl bedeuten.

8. 2-Hydroxynaphthalin nach Anspruch 7, dadurch gekennzeichnet, dass $R_1$ und $R_3$ Wasserstoff und $R_2$ und $R_4$ gleich oder verschieden sind und durch mindestens eine Methyl- oder Trifluormethylgruppe und/oder durch Chlor substituiertes Phenyl bedeuten.

9. Diazotypiematerial mit mindestens einer Diazoverbindung in der lichtempfindlichen Schicht, dadurch gekennzeichnet, dass es als Kupplungskomponente mindestens ein 2-Hydroxynaphthalin der allgemeinen Formel nach Anspruch 1 enthält.

10. Material nach Anspruch 9, dadurch gekennzeichnet, dass das Molverhältnis von Diazoverbindung zu Kupplungskomponente (1-1,2) : 1 beträgt.

## Claims

1. Derivatives of 2-hydroxynaphthalene of the general formula

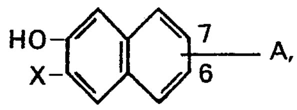

wherein

A in the 6 or 7-position is 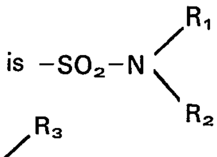

and

X is −OH or 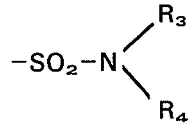

$R_1$ and $R_3$ are hydrogen, $R_2$ and $R_4$ are identical or different and are cycloalkyl having up to 8 carbon atoms, which is optionally substituted by lower alkyl groups, aralkyl having up to 10 carbon atoms, or aryl which is substituted by lower alkyl, lower alkoxy, halogen-substituted amino groups or by halogen, or $R_1$ and $R_2$ or $R_3$ and $R_4$ in each case are, conjointly with the nitrogen atom to wich they are linked, identical or different heterocyclic groups which are optionally substituted by lower alkyl.

2. A 2-hydroxynaphthalene as claimed in claim 1, wherein $R_2$ and $R_4$ are identical or different and are cycloalkyl having up to 7 carbon atoms, aralkyl having up to 10 carbon atoms or aryl having up to 10 carbon atoms, which is substituted by alkyl, alkoxy, halogen-substituted alkyl or dialkylamino groups having up to 4 carbon atoms or by halogen, or $R_1$ and $R_2$ or $R_3$ and $R_4$ are, conjointly with the nitrogen atom to which they are linked, pyrrolidine, piperidine, piperazine, morpholine, thiomorpholine or hexamethyleneimine.

3. A 2-hydroxynaphthalene as claimed in claim 1, wherein X− is a hydroxyl group.

4. A 2-hydroxynaphthalene as claimed in claims 1 and 3, wherein A is a sulfonamide group, with $R_1$ being hydrogen and $R_2$ being phenyl which is substituted by at least one lower alkyl, lower alkoxy, halogen-substituted alkyl or dialkylamino group and/or by halogen.

5. A 2-hydroxynaphthalene as claimed in claim 4, wherein $R_1$ is hydrogen and $R_2$ is phenyl which is substituted by at least one methyl or trifluoromethyl group and/or by chlorine.

6. A 2-hydroxynaphthalene as claimed in claim 1, wherein X− is a sulfonamide group.

7. A 2-hydroxynaphthalene as claimed in claims 1 and 6, wherein $R_1$ and $R_3$ are hydrogen and $R_2$ and $R_4$ are identical or different and are phenyl which is substituted by at least one lower alkyl, lower alkoxy, halogen-substituted alkyl or dialkylamino group and/or by halogen.

8. A 2-hydroxynaphthalene as claimed in claim 7, wherein $R_1$ and $R_3$ are hydrogen and $R_2$ and $R_4$ are identical or different and are phenyl which is substituted by at least one methyl or trifluoromethyl group and/or by chlorine.

9. A diazotype material comprising at least one diazo compound in its light-sensitive layer, wherein the coupling component used is at least one 2-hydroxynaphthalene of the general formula according to claim 1.

10. A material as claimed in claim 9, wherein the molar ratio of diazo compound to coupling component ranges from (1-1.2) : 1.

**Revendications**

1. Dérivés du 2-hydroxynaphtalène de formule générale

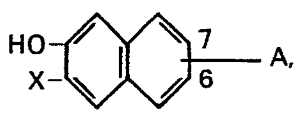

dans laquelle

A en position 6 ou 7 est le groupe

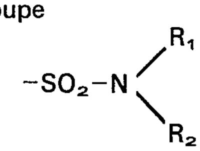

et

X est −OH ou le groupe 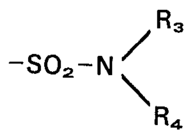

et $R_1$ et $R_3$ sont de l'hydrogène, $R_2$ et $R_4$, identiques ou différents, sont des groupes cycloalkyle ayant jusqu'à 8 atomes de carbone, éventuellement substitués par des groupes alkyle inférieur, des groupes aralkyle ayant jusqu'à 10 atomes de carbone ou des groupes amino substitués par des groupes alkyle inférieur, alcoxy inférieur, halogénoalkyle ou alkyle ou des groupes aryle substitués par de l'halogène, ou bien $R_1$ et $R_2$ ou $R_3$ et $R_4$ forment ensemble avec l'atome d'azote, auquel ils sont liés, des restes hétérocycliques, identiques ou différents, éventuellement substitués par de l'alkyke inférieur.

2. 2-hydroxynaphtalène selon la revendication 1, caractérisé en ce que $R_2$ et $R_4$ sont identiques ou différents et représentent des groupes cycloalkyle ayant jusqu'à 7 atomes de carbone, des groupes aralkyle ayant jusqu'à 10 atomes de carbone, ou bien des goupes aryle ayant jusqu'à 10 atomes de carbone substitués par des groupes alkyle, alcoxy, halogénoalkyle ou dialkylamino ayant jusqu'à 4 atomes de carbone, ou par de l'halogène, ou bien $R_1$ et $R_2$ ou $R_3$ et $R_4$ forment ensemble avec l'atome d'azote auquel ils sont liés les noyaux suivants: pyrrolidine, pipéridine, pipérazine, morpholine, thiomorpholine ou hexaméthylène-imine.

3. 2-hydroxynaphtalène selon la revendication 1, caractérisé en ce que X− est un groupe hydroxyle.

4. 2-hydroxynaphtalène selon les revendications 1 et 3, caractérisé en ce que A est un reste sulfonamide, $R_1$ étant de l'hydrogène, et $R_2$ un groupe phényle substitué par au moins un groupe alkyle inférieur, alcoxy inférieur, halogénoalkyle, ou dialkylamino et/ou par de l'halogène.

5. 2-hydroxynaphtalène selon la revendication 4, caractérisé en ce que $R_1$ est de l'hydrogène et $R_2$ un groupe phényle substitué par au moins un groupe méthyle ou trifluorométhyle et/ou par du chlore.

6. 2-hydroxynaphtalène selon la revendication 1, caractérisé en ce que X− est un groupe sulfonamide.

7. 2-hydroxynaphtalène selon les revendications 1 et 6, caractérisé en ce que $R_1$ et $R_3$ sont de l'hydrogène et $R_2$ et $R_4$, identiques ou différents, sont des groupes phényle substitués par au moins un groupe alkyle inférieur, alcoxy inférieur, halogénoalkyle ou dialkylamino et/ou par de l'halogène.

8. 2-hydroxynaphtalène selon la revendication 7, caractérisé en ce que $R_1$ et $R_3$ sont de l'hydrogène, $R_2$ et $R_4$ sont identiques, ou différents, et sont des groupes phényle substitués par au moins un groupe méthyle ou trifluorométhyle et/ou par du chlore.

9. Matériel pour diazotypie comprenant au moins un composé diazoïque dans la couche photosensible, caractérisé en ce qu'il contient comnme agent de copulation au moins un 2-hydroxynaphtalène de la formule générale définie dans la revendication 1.

10. Matériel selon la revendication 9, caractérisé en ce que le rapport molaire entre le composé diazoïque et l'agent de copulation est compris entre (1-1,2) : 1.

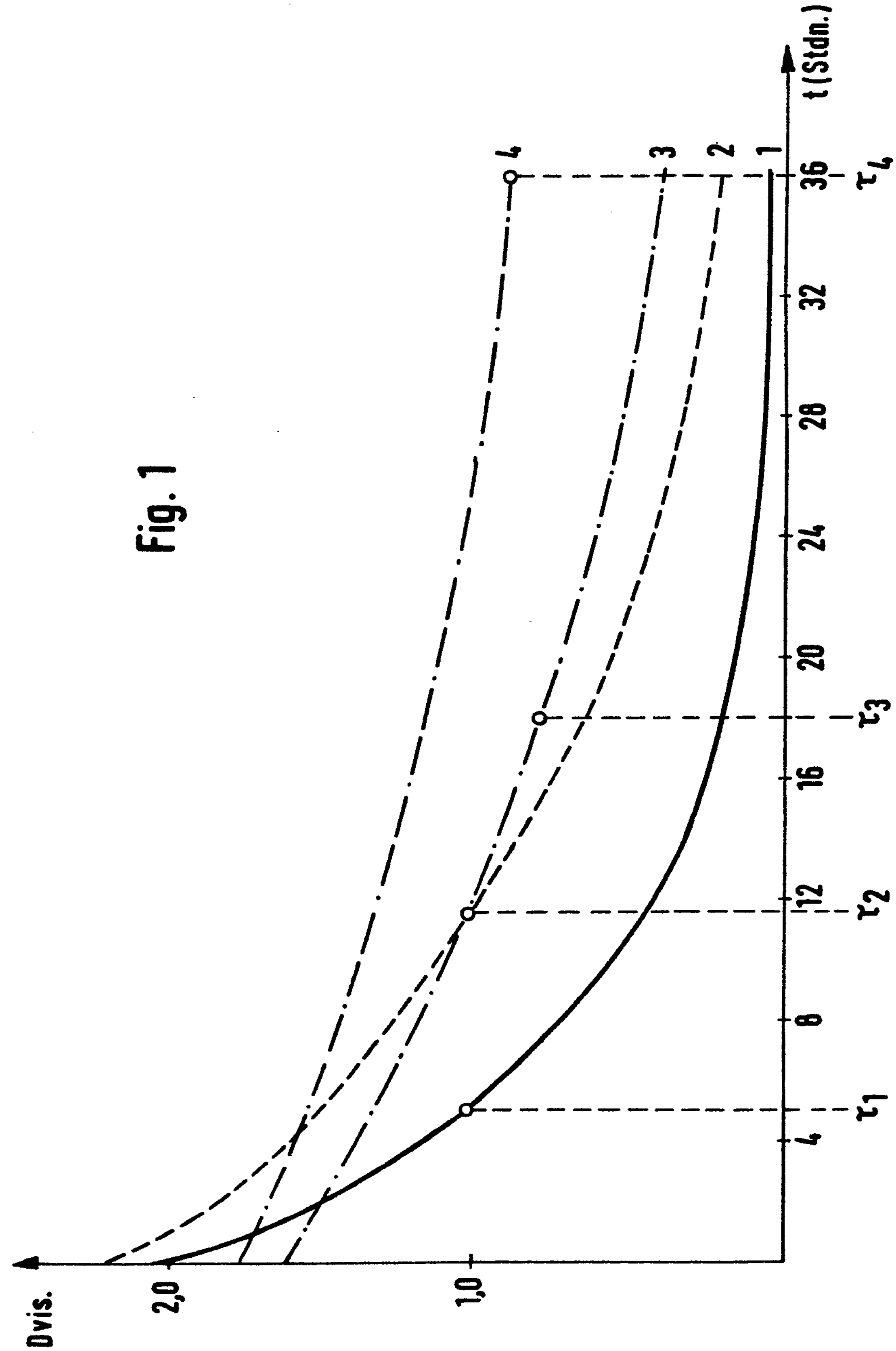


Fig. 1